# EUROPEAN PATENT APPLICATION

(11) **EP 1 790 345 A1**
(43) Date of publication of application: **30.05.2007**
(21) Application number: 05023893.0
(22) Date of filing: 02.11.2005
(51) Int. Cl.: A61K 31/53, A61K 31/341, A61P 25/28, A61K 31/4453, A61K 31/5377

(54) **Triazin beta-secretase inhibitors**

(71) Applicant: Esbatech AG, 8952 Schlieren (CH)
(72) Inventor: Lüthi, Urs, 8330 Pfäffikon (CH); Danzhi, Huang, 8051 Zürich (CH); Kolb, Peter, 8057 Zürich (CH); Cecchini, Marco, Allée Gaspard Mange 67000 Strasbourg (CH); Majeux, Nicolas, 8051 Zürich (CH); Dey, Fabian, 8055 Zürich (CH); Audédat, Stephan Valentin, 8107 Buchs (CH); Caflisch, Amedeo, 8057 Zürich (CH); Barberis, Alcide, 8057 Zürich (CH)
(74) Representative: Blum, Rudolf Emil

(57) **Abstract**

It has been found that compounds of formula (I) 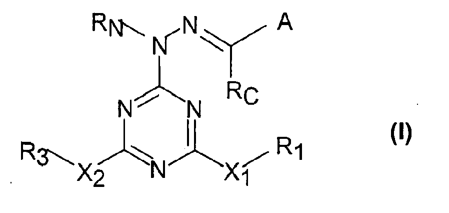 wherein, in preferred embodiments
X₁ and X₂ are N-H or N-R₃ or N-R₄,
R_{N} and R_{C} are both H
R₁ and R₃ are independently from each other optionally substituted phenyl, or R₁ and R₂ and/or R₃ and R₄ form together with the N to which R2 and/or R₄, respectively, are bound a 5 or 6 membered heterocycle comprising 1 or 2 heteroatoms, and
A is preferably halogen substituted phenyl or a preferably phenyl substituted furanyl
are good β-secretase inhibitors for the treatment of Alzheimer's disease.

## Description

### Field of the Invention

This invention relates to compounds acting as beta-secretase inhibitors.

### Background Art

Alzheimer's disease (AD) is the most common form of dementia among older people, and affects parts of the brain that control thought, memory and language. Susceptibility to Alzheimer's disease increases with age, but Alzheimer's disease is not a normal part of the ageing process.

A characteristic of this disease is the presence of extracellular senile plaque, the major component of which is the β-amyloid peptide (Aβ). The hydrophobic, 39-43-amino-acid-long Aβ peptide is excised from the amyloid precursor protein (APP) by sequential cleavage by the so-called β- and γ-secretases.

Known genetic predispositions for AD mostly affect genes involved in Aβ generation or Aβ deposition. Since the Aβ peptide seems to play an important role in the pathogenesis of AD, current therapeutic strategies often focus on inhibition of Aβ deposition and generation. Inhibition of β-secretase activity represents an attractive option to achieve this goal.

Despite major efforts to identify novel β-secretase inhibitors by applying in vitro high-throughput screening (HTS) assays with purified soluble BACE-1 fragments and fluorogenic peptide substrates, the best progress towards efficient BACE-1 inhibition has been achieved so far by the use of peptidic transition-state mimetic compounds. However, for efficient inhibition of β-secretase in cells, their molecular weight must be reduced and their structure modified so as to allow for permeation of cellular membranes, the blood-brain barrier and for activity in the natural cellular environment.

There also exist some assays for identifying low molecular weight inhibitors of secretases that can block these membrane-bound enzymes at the natural location within intracellular compartments. Cell-based HTS assays, however, are generally faced with the problem that selection signals are often caused by compounds that interfere with cellular processes or pathways that are redundant with that of the target. For example, some compounds found by mammalian cell based assays impair the production of Aβ through the increase of the pH in intracellular compartments, or they function through protein phosphorylation, or they simply catalyze polymerization of Aβ, thus reducing the percentage of soluble peptide.

Some candidate compounds for inhibiting the production of Aβ peptide in a biological system have been proposed in US 5,814,646 and US 5,624,937, and triazine compounds have already been suggested for the treatment of Alzheimer's disease (see GB 2 397 301, WO 2004/063196).

Nevertheless, there is still a need for potent β-secretase inhibitors that directly inhibit β-secretase.

### Disclosure of the Invention

Hence, it is a general object of the invention to provide compounds that directly act as β-secretase inhibitors.

Now, in order to implement these and still further objects of the invention, which will become more readily apparent as the description proceeds, the β-secretase inhibitors of the present invention are manifested by the following formula I 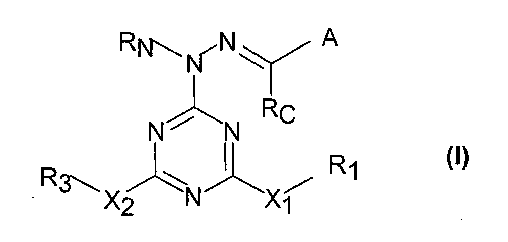 wherein
X₁ and X₂ independently from each other are selected from O or S or N-R₂ or N-R₄, and in particular X₁ is N-R₂ and X₂ is N-R₄,
R_{N} and R_{C} are independently from each other chosen from small substituents such as -H, -OH, -CH=O, NH₂, preferably -H,
R₁ and R₃ are independently from each other selected from the group comprising C1-C3 alkoxy, in particular C1-C3 alkoxy, 3- to 8-membered aliphatic or heteroaliphatic cycles, optionally substituted 5- or 6-membered heteroaryl or aryl, in particular optionally substituted phenyl, with the substituents being independently from each other selected from 1 to 5, in particular 1 or 2, of C1-C6 alkyl, halogen, C1-C6 alkoxy, hydroxy and C2-C6 alkene, wherein preferred alkyls are C1-C3 alkyl, in particular methyl, the halogens are selected from fluorine, chlorine, bromine and iodine, in particular from fluorine and chlorine, preferred alkoxy are C1-C3 alkyloxy, in particular methoxy,
R₂ and R₄ are independently from each other selected from the group comprising hydrogen, C1-C3 alkyl, C1-C3 alkoxy, phenoxy, C1-C3 thioalkyl, wherein preferred C1-C3 alkyl is methyl, preferred C1-C3 alkoxy is methoxy, and preferred C1-C3 thioalkyl is -S-CH₃,
or R₁ and R₂ and/or R₃ and R₄ form together with the N to which R2 and/or R₄, respectively, are bound a 5 or 6 membered heterocycle comprising 1 or 2 heteroatoms, the second heteroatom being N or O, in particular 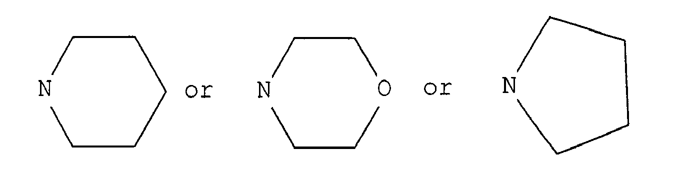 A is 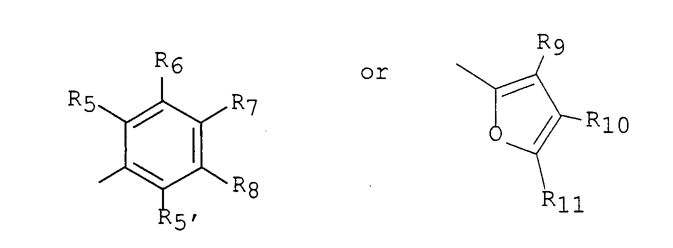 wherein
R₅ is selected from the group comprising -H, -OH, -CH₃, -CH₂OH, -CH₂CH₃, -CH₂CH₂CH₃, -NH-CH₂CH₃,
R₅, is selected from the group comprising -H, -OH, -CH₃, -CH₂OH, -CH₂CH₃, -CH₂CH₂CH₃, -NH-CH₂CH₃, -O-CH₂CH₃, -O-phenyl, -CH₂-phenyl, -NH-phenyl, -F, -Cl, -Br, -I, and much preferred is -H,
R₆ is selected from the group comprising -H, -F, -Cl, -Br, -I,
R₇ is selected from the group comprising -H, -OH, -CH₃, -CH₂OH, -CH₂CH₃, -CH₂CH₂CH₃, -NH-CH₂CH₃, -O-CH₂CH₃, -O-phenyl, -CH₂-phenyl, -NH-phenyl,
R₈ is selected from the group comprising -H, -F, -Cl, -Br, -I, and wherein
R₉ and R₁₀ are independently from each other selected from the group comprising -H, -CH₃, -CH₂CH₃, -CH₂CH₂CH₃, phenyl, -CH₂-phenyl, -O-phenyl, -NH-phenyl, -Br
R₁₁ is selected from the group comprising -CH₂CH₂CH₂COOH, -CH₂CH₂COOH, -NH-CH₂CH₂COOH, -NH-CH₂COOH, -O-CH₂CH₂COOH, -O-CH₂COOH, -S-CH₂CH₂COOH, -S-CH₂COOH, -Br, and 5- or 6-membered heteroaromatic or aromatic rings, in particular 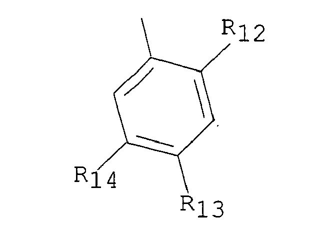 wherein R₁₂ is selected from the group comprising -H, halogen selected from -Cl, -Br, -I, in particular -Cl, C1-C3 alkyl, in particular -CH₃, -OH, C1-C3 alkoxy, in particular methoxy,
R₁₃ is selected from the group comprising -H, -OH, -NO₂, -Cl, -Br, -I, in particular -Cl, C1-C3 alkyl, in particular -CH₃, C1-C3 alkoxy, in particular methoxy,
R₁₄ is selected from the group comprising -H, -COOH, -COOR with R being selected from C1-C6 alkyl or C2-C6 alkenyl, -NO₂, -Cl, -(C=O)CH₃, SO₂,
or pharmaceutically acceptable salts thereof.

In preferred embodiments, at least one of the below mentioned preferences is present, preferably two or more of them, most preferred all of them.
(i) X₁ is N-R₂ and X₂ is N-R₄
(ii) at least one of R₁ and R₃ are optionally substituted phenyl with the substituents being independent from each other selected from 1 or 2 of C1-C3 alkyl, in particular methyl; halogen selected from chlorine, bromine and iodine, in particular fluorine and chlorine; C1-C3 alkyloxy, in particular methoxy, and
   R₂ and R₄ are hydrogen,
(iii) R₁ and R₂ and/or R₃ and R₄ form together with the N to which R2 and/or R₄, respectively, are bound a 5 or 6 membered heterocycle comprising 1 or 2 heteroatoms, the second heteroatom being N or O, in particular 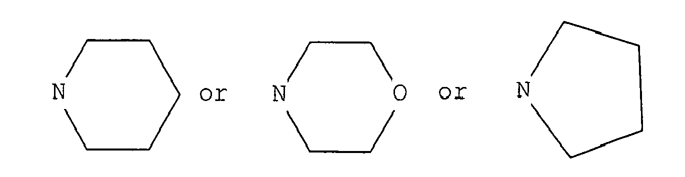
(iv) at most one of R₁ or R₃ is C1-C3-alkoxy, in particular methoxy
(v) A is 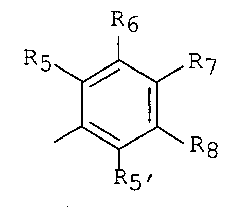 wherein
   R₅ is -H or -OH,
   R₅, is -H,
   R₆ is selected from the group comprising -H, -Cl, -Br, -I, -OCH₃,
   R₇ is -H or -OH,
   R₈ is selected from the group comprising H, Cl, Br, I, or
(vi) A is 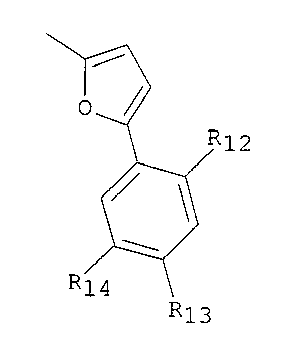 wherein
   R₁₂ is selected from the group comprising -H, -Cl, -CH₃,
   R₁₃ is selected from the group comprising -H, -OH, -Cl, -NO₂,
   R₁₄ is selected from the group comprising -H, -COOH, -NO₂, -Cl,
(vii) R_{N} and R_{C} are both -H.

Much preferred are compounds wherein X₁ is N-R₂, X₂ is N-R₄, and R_{N} and R_{C} are both -H, and which additionally have at least one of the preferences below, namely
(i) R₁ and R₃ are optionally substituted phenyl with the substituents being independent from each other selected from 1 or 2 of methyl, fluorine, chlorine, and methoxy, and
   R₂ and R₄ are hydrogen,
(ii) R₁ and R₂, and R₃ and R₄ form together with the N to which R2 and R₄ are bound a heterocycle selected from 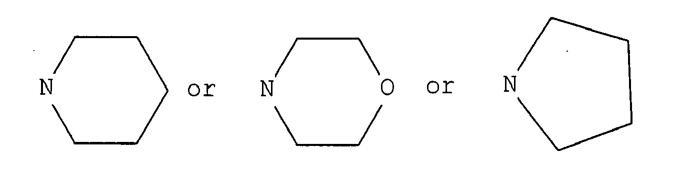
(iii) R₁ is optionally substituted phenyl with the substituents being independent from each other selected from 1 or 2 of methyl, fluorine, chlorine, and methoxy, and R₂ is hydrogen, and R₃ and R₄ form together with the N to which R₄ is bound a heterocycle selected from 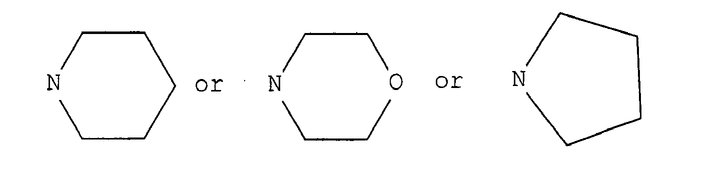
(iii) R₁ is as defined in (i) or (ii) and R₃ is methoxy
(iv) A is 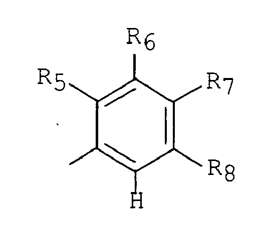 wherein at least one of R₆ and R₈ is Cl, Br, I, preferably both, and much preferred both are the same,
(v) A is 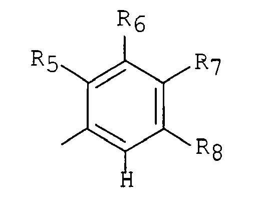 wherein at least 3 of R₅ to R₈ are not hydrogen, preferably at least R₈ and much preferred R₈ and R₆ are Cl, Br or I , in particular R₈ and R₆ are the same
(vi) A is 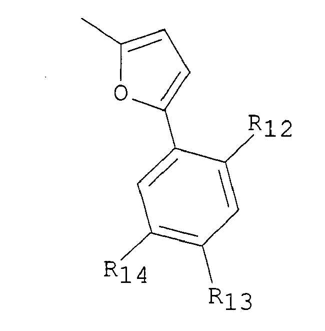 wherein R₁₄ is -COOH,
(vii) R₁₄ is -COOH and R₁₃ is -OH or -Cl (viii)R₁₃ is -OH or -Cl or -NO₂.

It has been found that compounds of the above formulas are efficient in inhibiting β-secretase activity. Thus, such compounds are suitable in the treatment and prophylaxis of β-secretase activity related diseases or diseases related to the deposition of amyloid beta-protein, respectively, such as Alzheimer's disease, Down's syndrome, and advanced aging of brain.

In therapeutic applications, the compounds are administered to a host already suffering from the disease. The compounds will be administered in an amount sufficient to inhibit further deposition of senile plaques. The specific dose of compound(s) administered according to this invention to obtain therapeutic and/or prophylactic effects will, of course, be determined by the particular circumstances, such as the specific compound administered, the condition being treated, etc. A daily dose will contain a dosage level of from about 0.01 mg/kg to about 50 mg/kg of body weight of an active compound, preferably from about 0.05 mg/kg to about 20 mg/kg, for example from about 0.1 mg/kg to about 120 mg/kg.

The compound can be administered by a variety of routes including oral, rectal, transdermal, subcutaneous, intravenous, intramuscular and intranasal either as such, but preferable in a formulation comprising carriers adjuvants etc. Suitable pharmaceutically acceptable solid and liquid carriers and/or pharmaceutically acceptable adjuvants, such as stabilizing agents, emulsifyers, etc. are known in the art.

For example, a typical pharmaceutical composition for intramuscular injection would contain about one µg to one mg of the compound in from one to four milliliters of sterile buffered water. The typical pharmaceutical composition for intravenous infusion would contain about one to one hundred milligrams of the compound in from one hundred to five hundred milliliters of sterile Ringer's solution.

The pharmaceutical formulations are prepared by known procedures using known and readily available ingredients.

### Modes for Carrying Out the Invention

Specific compounds and their β-secretase inhibiting effects are further described below.

The compounds investigated were the compounds with the following formulas:

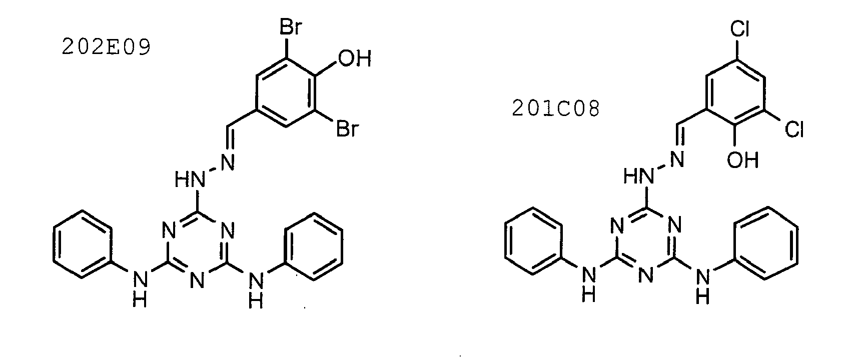

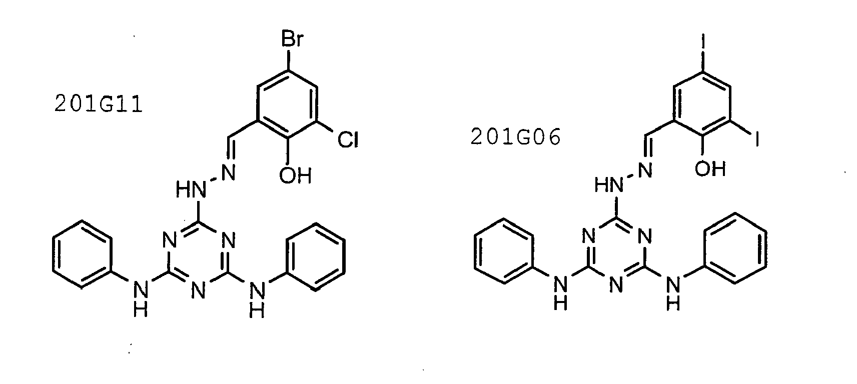

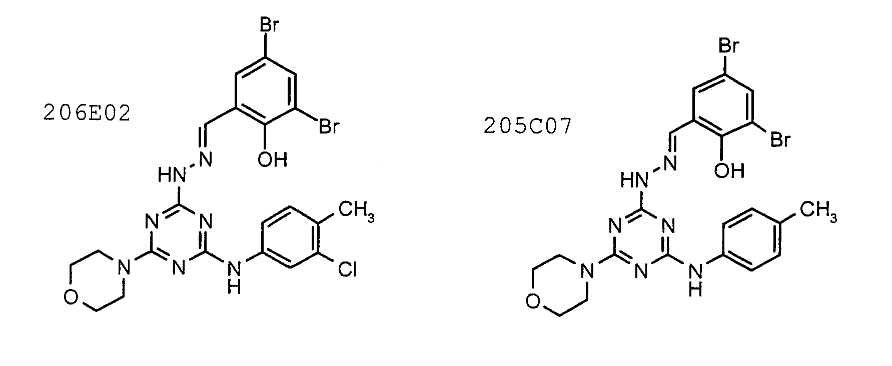

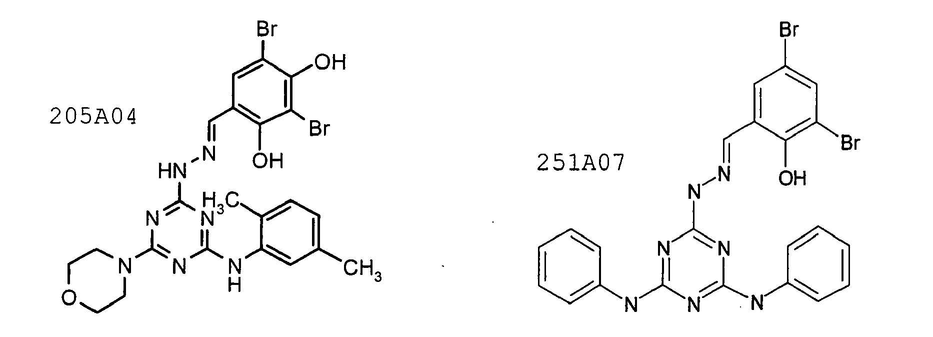

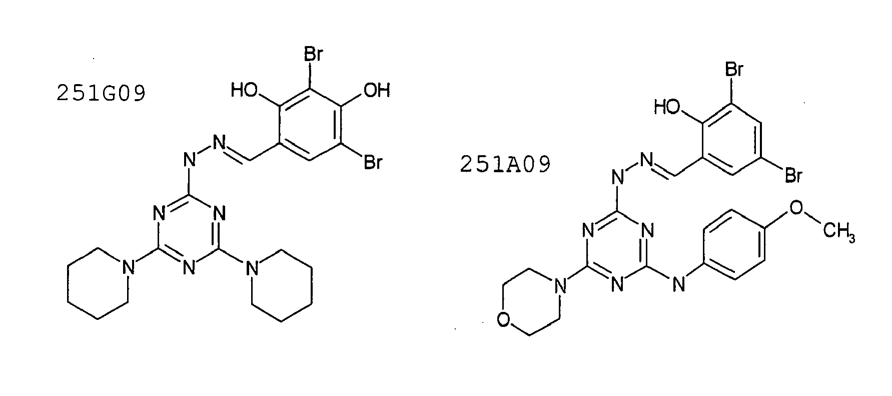

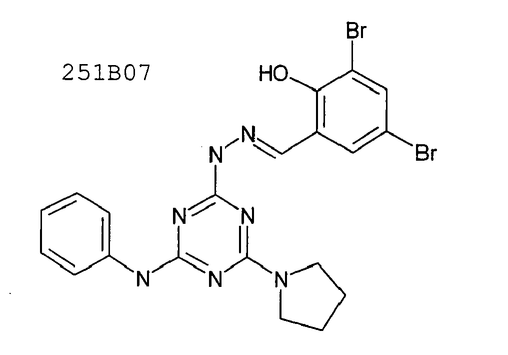

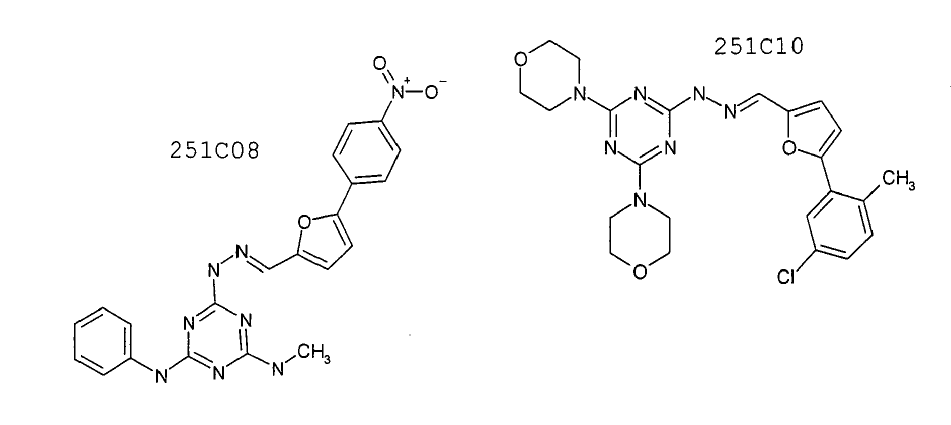

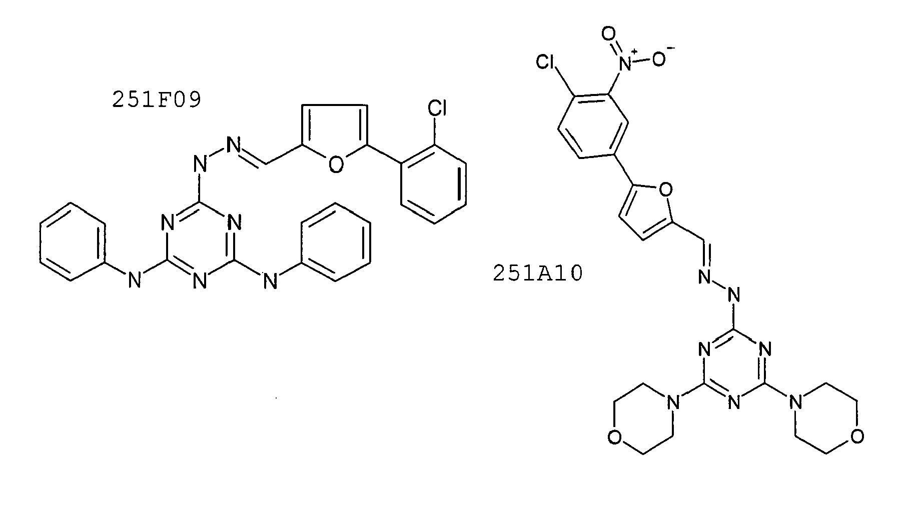

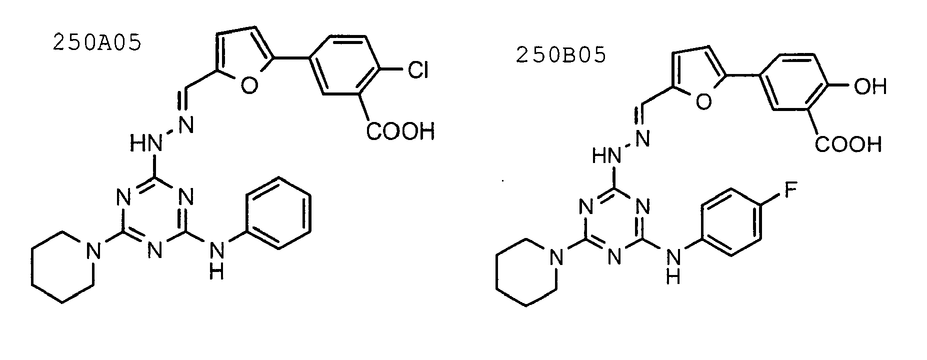

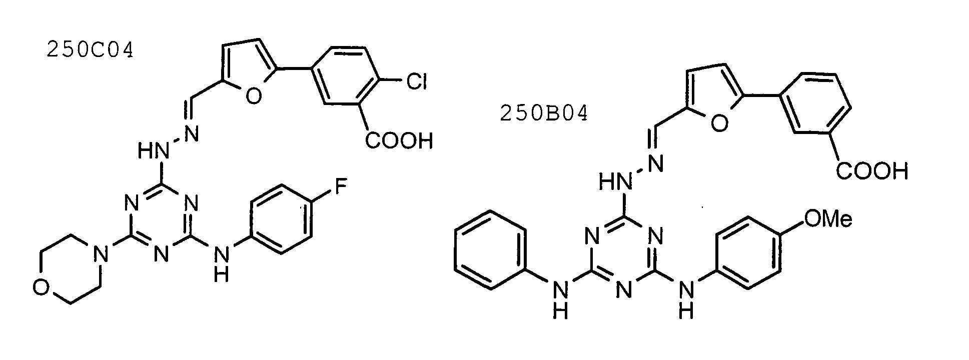

The tests performed were
a) Aβ1-40 (Sw) bioassay, which measures the amount of the amyoid peptide Aβ1-40 in the supernatant of Swedish APP695 transgenic HEK293 cells in the presence of the various BACE inhibitors via ELISA (enzyme-linked immunosorbent assay). In the table, the inhibitory concentration that reduces Aβ1-40 secretion to 50 % is indicated (IC50), or the % reduction of Aβ1-40 secretion at the indicated concentration.
b) FRET assay, which measures the activity of recombinant BACE enzyme in the presence of the various BACE inhibitors via a FRET (fluorescence resonance energy transfer)-based readout. In the table, the inhibitory concentration that reduces the activity of BACE to 50 % is indicated (IC50), or the % reduction of the activity of BACE at the indicated concentration.
c) An additional in silico test was performed for the compounds listed in Tables 1 to 3. The compounds were docked with the FFLD approach (Budin et al., Biol. Chem. 382, 1365-1372, 2001) and their binding energy was evaluated with the LIECE method (Huang and Caflisch, J. Med. Chem. 47, 5791-5797, 2004). The affinity evaluated with LIECE is in the low micromolar range for most of these compounds.

**Table 1:**

| **Compound** | **LIECE Ki [µM]** | **Aβ1-40(sw) bioassay (cell-based)** | **BACE FRET assay (in vitro)** |
|---|---|---|---|
| 202E09 | 41.41 | 25% (5 µM) | IC₅₀ 53.9 µM |
| 201C08 | 20.20 | 25% (5 µM) | IC₅₀ 11.2 µM |
| 201G11 | 74.22 | 26% (5 µM) | IC₅₀ 11.9 µM |
| 201G06 | 38.77 | No (5 µM) | IC₅₀ 25.5 µM |
| 206E02 | 49.35 | 27% (50 µM) | IC₅₀ 20.6 µM |
| 205C07 | 30.89 | No (50 µM) | IC₅₀ 48.1 µM |
| 205A04 | 56.49 | No (5 µM) | IC₅₀ 81.8 µM |
| 251A07 | 13.00 | not done | IC₅₀ 10.6 µM |
| 251C08 | 20.00 | not done | IC₅₀ 24.4 µM |
| 251A09 | 14.00 | not done | IC₅₀ 34.4 µM |
| 251G09 | 24.00 | not done | IC₅₀ 169.7 µM |
| 251B07 | 12.00 | not done | IC₅₀ 45.4 µM |
| 251C10 | -- | not done | IC₅₀ >100 µM |
| 251F09 | 15.00 | not done | IC₅₀ 188.5 µM |
| 251A10 | 18.00 | not done | IC₅₀ 123.7 µM |
| 250A05 | 6.64 | IC₅₀ ~10-20 µM | IC₅₀ 141.5 µM |
| 250B05 | 2.68 | IC₅₀ ~10-20 µM | IC₅₀ 30.9 µM |
| 250C04 | 12.82 | 0 (25 µM) | IC₅₀ 7.1 µM |
| 250B04 | 12.61 | IC₅₀ ~10-20 µM | IC₅₀ 66.6 µM |

While there are shown and described presently preferred embodiments of the invention, it is to be distinctly understood that the invention is not limited thereto but may be otherwise variously embodied and practiced within the scope of the following claims.

## Claims

1. A β-secretase inhibitor of formula I 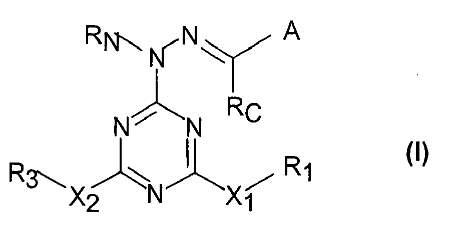 wherein
X₁ and X₂ independently from each other are selected from O or S or N-R₂ or N-R₄,
R_{N} and R_{C} are independently form each other selected from small substituents selected from -H, -OH, - CHO, -NH₂,
R₁ and R₃ are independently from each other selected from the group consisting of C1-C6 alkoxy, 3- to 8-membered aliphatic or heteroaliphatic cycles, optionally substituted 5- or 6-membered heteroaryl or aryl, in particular optionally substituted phenyl, with the substituents being independent from each other selected from 1 to 5, preferably from 1 or 2, of C1-C6 alkyl, halogen selected from fluorine, chlorine, bromine, iodine, C1-C6 alkyloxy, hydroxy, C1-C6 alkene,
R₂ and R₄ are independently from each other selected from the group consisting of hydrogen, C1-C3 alkyl, C1-C3 alkoxy, phenoxy, C1-C3 thioalkyl,
or R₁ and R₂ and/or R₃ and R₄ form together with the N to which R2 and/or R₄, respectively, are bound a 5 or 6 membered heterocycle comprising 1 or 2 heteroatoms, the second heteroatom being N or O,
A is 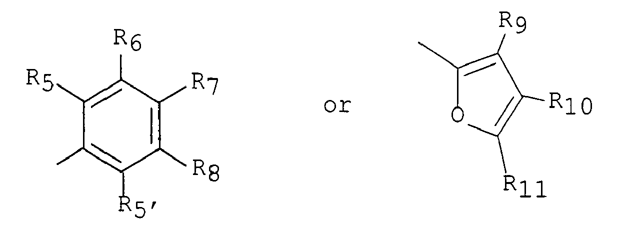 wherein
R₅ is selected from the group consisting of -H, -OH, -CH₃, -CH₂OH, -CH₂CH₃, -CH₂CH₂CH₃, -NH-CH₂CH₃,
R_{5'} is selected from the group consisting of -H, -OH, -CH₃, -CH₂OH, -CH₂CH₃, -CH₂CH₂CH₃, -NH-CH₂CH₃, -O-CH₂CH₃, -O-phenyl, -CH₂-phenyl, -NH-phenyl, -F, -Cl, -Br, -I,
R₆ is selected from the group consisting of -H, -F, -Cl, -Br, -I,
R₇ is selected from the group consisting of -H, -OH, -CH₃, -CH₂OH, -CH₂CH₃, -CH₂CH₂CH₃, -NH-CH₂CH₃, -O-CH₂CH₃, -O-phenyl, -CH₂-phenyl, -NH-phenyl,
R₈ is selected from the group consisting of -H, -F, -Cl, -Br, -I, and wherein
R₉ and R₁₀ are independently from each other selected from the group consisting of -H, -CH₃, -CH₂CH₃, -CH₂CH₂CH₃, phenyl, -CH₂-phenyl, -O-phenyl, -NH-phenyl, Br
R₁₁ is selected from the group consisting of -CH₂CH₂CH₂COOH, -CH₂CH₂COOH, -NH-CH₂CH₂COOH, -NH-CH₂COOH, -O-CH₂CH₂COOH, -O-CH₂COOH, -S-CH₂CH₂COOH, -S-CH₂COOH, -Br, and 5- and 6-membered heteroaromatic or aromatic rings, in particular 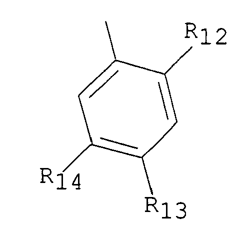 wherein R₁₂ is selected from the group consisting of -H, -Cl, -Br, -I, C1-C3 alkyl, in particular -CH₃, -OH, C1-C3 alkoxy, in particular methoxy
R₁₃ is selected from the group consisting of -H, -OH, -NO₂, -Cl, -Br, -I, C1-C3 alkyl, in particular - CH₃, C1-C3 alkoxy, in particular methoxy and
R₁₄ is selected from the group consisting of -H, -COOH, -COOR, wherein R is selected from C1-C6 alkyl or C2-C6 alkenyl, -NO₂, Cl, -(C=O)-CH₃, SO₂,
or pharmaceutically acceptable salts thereof
as pharmaceutical.

2. The β-secretase inhibitor of claim 1 wherein X₁ is N-R₂ and X₂ is N-R₄.

3. The β-secretase inhibitor of anyone of the preceding claims, wherein R₁ and R₃ are independently from each other selected from the group consisting of methoxy, optionally substituted phenyl with the substituents being independent from each other selected from 1 or 2 of methyl fluorine, chlorine, and methoxy, and/or R₂ and R₄ are independently from each other selected from the group consisting of hydrogen, methoxy, phenoxy,-S-CH₃, and/or R₁ and R₂ and/or R₃ and R₄ form together with the N to which R2 and/or R₄, respectively, are bound a 5 or 6 membered heterocycle selected from

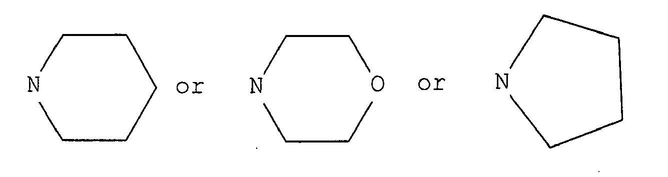

4. The β-secretase inhibitor of anyone of the preceding claims, wherein
R₁₁ is 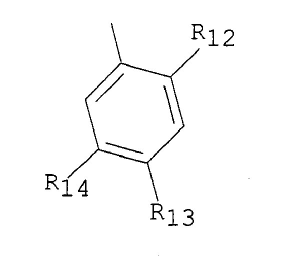 wherein R₁₂, R₁₃, and R₁₄ are as defined above.

5. The β-secretase inhibitor of anyone of the preceding claims, wherein R₁ and/or R₃ are optionally substituted phenyl with the substituents being independent from each other selected from 1 or 2 of C1-C3 alkyl, in particular methyl; halogen, in particular fluorine and chlorine; C1-C3 alkyloxy, in particular methoxy.

6. The β-secretase inhibitor of anyone of the preceding claims, wherein R₁ and R₃ are optionally substituted phenyl with the substituents being independent from each other selected from 1 or 2 of methyl, fluorine, chlorine, and methoxy

7. The β-secretase inhibitor of anyone of the preceding claims, wherein R₂ and R₄ are hydrogen.

8. The β-secretase inhibitor of anyone of the preceding claims, wherein R₁ and R₂ and/or R₃ and R₄ form together with the N to which R₂ and/or R₄, respectively, are bound a 5 or 6 membered heterocycle comprising 1 or 2 heteroatoms, the second heteroatom being N or O, in particular

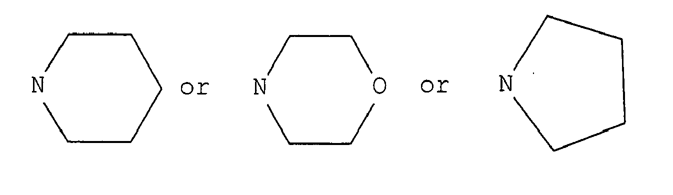

9. The β-secretase inhibitor of anyone of the preceding claims, wherein R₁ and R₂, and R₃ and R₄ form together with the N to which R2 and R₄ are bound a heterocycle selected from

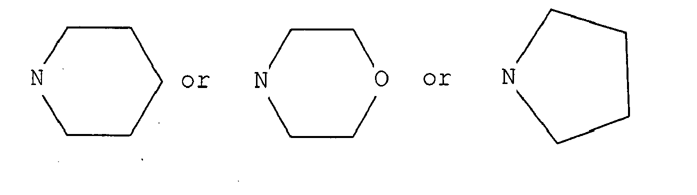

10. The β-secretase inhibitor of anyone of the preceding claims, wherein R₁ is optionally substituted phenyl with the substituents being independent from each other selected from 1 or 2 of methyl, fluorine, chlorine, and methoxy, and R₂ is hydrogen, and R₃ and R₄ form together with the N to which R₄ is bound a heterocycle selected from

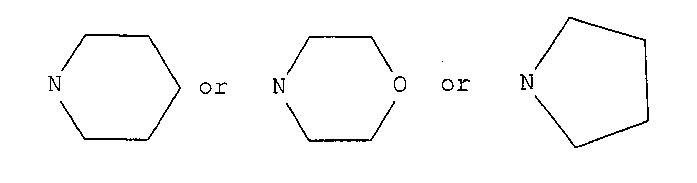

11. The β-secretase inhibitor of anyone of the preceding claims, wherein at most one of R₁ or R₃ is C1-C3-alkoxy, in particular methoxy.

12. The β-secretase inhibitor of claim 11, wherein R₃ is methoxy.

13. The β-secretase inhibitor of anyone of the preceding claims, wherein A is 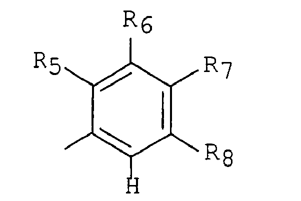 wherein
R₅ is -H or -OH,
R₆ is selected from the group consisting of -H, -Cl, -Br, -I, -OCH₃,
R₇ is -H or -OH,
R₈ is selected from the group consisting of H, Cl, Br, I.

14. The β-secretase inhibitor of claim 13, wherein at least one of R₆ and R₈ is Cl, Br, I, preferably both, and much preferred both are the same.

15. The β-secretase inhibitor of anyone of the preceding claims, wherein at least 3 of R₅ to R₈ are not hydrogen, preferably at least R₈ and much preferred R₈ and R₆ are Cl, Br or I , in particular R₈ and R₆ are the same

16. The β-secretase inhibitor of anyone of the preceding claims, wherein A is 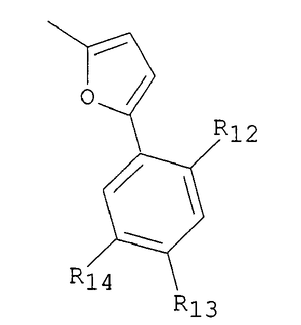 and wherein
R₁₂ is selected from the group consisting of -H, -Cl, -CH₃,
R₁₃ is selected from the group consisting of -H, -OH, -Cl, -NO₂,
R₁₄ is selected from the group consisting of -H, -COOH, -NO₂, Cl.

17. The β-secretase inhibitor of claim 16, wherein R₁₄ is -COOH.

18. The β-secretase inhibitor of claim 17, wherein R₁₃ is -OH or Cl.

19. Use of a β-secretase inhibitor of anyone of the preceding claims, or a pharmaceutically acceptable salt thereof, in the manufacture of a medicament for inhibiting the production and/or the accumulation of amyloid beta-protein in warm blooded mammals, in particular human beings, especially for the treatment of Alzheimer's disease and or Down's syndrome and/or aging of brain.

20. A pharmaceutical composition comprising at least one β-secretase inhibitor of anyone of claims 1 to 18, or a pharmaceutically acceptable salt thereof, together with a pharmaceutically acceptable carrier and optionally one or more adjuvants.
